(19) Europäisches Patentamt
European Patent Office
Office européen des brevets

(11) **EP 3 307 160 B1**

(12) **EUROPEAN PATENT SPECIFICATION**

(45) Date of publication and mention of the grant of the patent:
**26.07.2023 Bulletin 2023/30**

(21) Application number: **16728109.6**

(22) Date of filing: **27.05.2016**

(51) International Patent Classification (IPC):
*A61B 5/11* (2006.01)    *A61B 5/00* (2006.01)

(52) Cooperative Patent Classification (CPC):
**A61B 5/1118; A61B 5/4857; A61B 5/681; A61B 5/7278**

(86) International application number:
**PCT/IB2016/053106**

(87) International publication number:
**WO 2016/198985 (15.12.2016 Gazette 2016/50)**

(54) **SYSTEM AND METHOD FOR ESTIMATING CIRCADIAN PHASE**

SYSTEM UND VERFAHREN ZUR BERECHNUNG EINER BIORHYTHMUSPHASE

SYSTÈME ET PROCÉDÉ D'ESTIMATION D'UNE PHASE CIRCADIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2015 US 201562174162 P**

(43) Date of publication of application:
**18.04.2018 Bulletin 2018/16**

(73) Proprietor: **Koninklijke Philips N.V.**
**5656 AG Eindhoven (NL)**

(72) Inventor: **AUBERT, Xavier Louis Marie Antoine**
**5656 AE Eindhoven (NL)**

(74) Representative: **Philips Intellectual Property & Standards**
**High Tech Campus 52**
**5656 AG Eindhoven (NL)**

(56) References cited:
**WO-A1-2013/132454    US-A1- 2014 187 871**

• **Sonia Ancoli-Israel ET AL: "The Role of Actigraphy in the Study of Sleep and Circadian Rhythms", SLEEP 2003;26(3), 1 May 2003 (2003-05-01), pages 342-392, XP055165785, Retrieved from the Internet: URL:http://www.aasmnet.org/Resources/Pract iceReviews/cpr_Actigraphy.pdf [retrieved on 2015-01-29]**
• **ST HILAIRE ET AL: "Addition of a non-photic component to a light-based mathematical model of the human circadian pacemaker", JOURNAL OF THEORETICAL BIOLOGY, ACADEMIC PRESS, LONDON, GB, vol. 247, no. 4, 23 July 2007 (2007-07-23), pages 583-599, XP022165696, ISSN: 0022-5193, DOI: 10.1016/J.JTBI.2007.04.001 cited in the application**
• **Megan E Jewett ET AL: "JOURNAL OF BIOLOGICAL RHYTHMS / December 1999 Jewett et al. / HUMAN CIRCADIAN PACEMAKER MODEL Revised Limit Cycle Oscillator Model of Human Circadian Pacemaker", , 1 December 1999 (1999-12-01), XP055293052, Retrieved from the Internet: URL:http://isites.harvard.edu/fs/docs/icb. topic59176.files/Due_Wk_03_Oct_4/Jewett_Re vised_limit_osc.pdf [retrieved on 2016-08-03] cited in the application**

EP 3 307 160 B1

**Description**

BACKGROUND

1. Field

The present disclosure pertains to a system and method for estimating the circadian phase of a subject, and, in particular, to using activity level measurements to estimate the circadian phase.

2. Description of the Related Art

The medical importance of an individual person's circadian rhythm is well-documented, and includes, among other purposes, diagnostic purposes. Light deficiency disorders may include, but are not limited to, Seasonal Affective Disorder (SAD), circadian sleep disorders, and circadian disruptions associated with, *e.g.,* jet-lag, shift-work, and/or other occupational conditions that may cause circadian disruptions.

Various models and methods may be used to determine and/or estimate a subject's circadian phase. Typically, determinations and/or estimations are made based on light exposure and/or other measurements during a preceding period, *e.g.* of one or more days. Once the circadian phase of a particular subject has been determined and/or estimated, the circadian phase may be adjusted as desired and/or recommended by, *e.g.,* light therapy and/or exogenous melatonin intake.

Document WO 2013/132454 discloses a method for generating information about a person's circadian rhythm in terms of a difference between a reference and a specific circadian time.

Sonia Ancoli-Israel et al. "The role of actigraphy in the study of sleep and circadian rhythms", SLEEP 2003;26(3), 1 May 2003, pages 342-392, discloses methods for using actigraphy for the measurement of sleep or rhythms.

SUMMARY

The invention is defined by the claims.

Accordingly, it is an object of one or more embodiments of the present invention to provide a system to estimate circadian phase of a subject. The system comprises a sensor and one or more physical processors. The sensor is configured to detect a motion of the subject and generate output signals conveying information related to an activity level of the subject based on said motion. The one or more physical processors are configured to determine a set of activity parameters related to the activity level of the subject, wherein the set of activity parameters is based on the generated output signals. The one or more physical processors are further configured to generate a set of estimated light exposure parameters based on the set of activity parameters using the equation:

$$LE(t) = \frac{1}{K} \cdot \left(activity(t)\right)^{\gamma} - c_0 , \qquad 1 < \gamma < 3$$

wherein LE(t) is an estimated light exposure parameter, activity(t) is an activity parameter and K, $\gamma$ and $c_0$ are constants; and estimate the circadian phase of the subject based on the set of estimated light exposure parameters, wherein the model is a pacemaker oscillator model, wherein the pacemaker oscillator model is described by a second-order differential equation:

$$\frac{d^2}{dt^2} x(t) - \mu \cdot (1 - 4x^2)\frac{d}{dt}x(t) + \left(\frac{2\pi}{\tau}\right)^2 x(t) = \frac{d}{dt}(B(t) + N(t))$$

wherein x(t) is a dependent variable that is assumed to vary analogously with the subject's core body temperature, $\mu$ is a stiffness factor, $\tau$ is an intrinsic period, B(t) is a photic driving term and N is a non-photic driving term, wherein the photic driving term B(t) includes the set of light exposure parameters, and wherein estimation of the circadian phase includes a substitution of the set of light exposure parameters included in the model with the generated set of estimated light exposure parameters.

It is yet another aspect of one or more embodiments of the present invention to provide a method to estimate circadian phase of a subject. The method comprises generating, by a sensor configured to detect a motion of the subject, output signals conveying information related to an activity level of the subject based on said motion; determining a set of activity parameters related to the activity level of the subject, wherein the set of activity parameters is based on the generated output signals; generating a set of estimated light exposure parameters based on the set of activity parameters using the equation:

$$LE(t) = \frac{1}{K} \cdot \big(activity(t)\big)^{\gamma} - c_0, \qquad 1 < \gamma < 3$$

wherein LE(t) is an estimated light exposure parameter, activity(t) is an activity parameter and K, $\gamma$ and $c_0$ are constants;; and estimating the circadian phase of the subject based on the set of estimated light exposure parameters, wherein the model is a pacemaker oscillator model, wherein the pacemaker oscillator model is described by a second-order differential equation:

$$\frac{d^2}{dt^2} x(t) - \mu \cdot (1 - 4x^2)\frac{d}{dt}x(t) + \left(\frac{2\pi}{\tau}\right)^2 x(t) = \frac{d}{dt}(B(t) + N(t))$$

wherein x(t) is a dependent variable that is assumed to vary analogously with the subject's core body temperature, $\mu$ is a stiffness factor, $\tau$ is an intrinsic period, B(t) is a photic driving term and N is a non-photic driving term, wherein the photic driving term B(t) includes the set of light exposure parameters, and wherein estimation of the circadian phase includes a substitution of the set of light exposure parameters included in the model with the generated set of estimated light exposure parameters.

It is yet another aspect of one or more examples not covered by the claimed invention to provide a system configured to estimate circadian phase of a subject. The system comprises means for generating output signals conveying information related to an activity level of the subject; means for determining a set of activity parameters related to the activity level of the subject, wherein the set of activity parameters is based on the generated output signals; means generating a set of estimated light exposure parameters based on the set of activity parameters; and means for estimating the circadian phase of the subject based on the set of estimated light exposure parameters.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

BRIEF DESCRIPTION OF THE DRAWINGS

FIG. 1 schematically illustrates a system configured to estimate circadian phase of a subject, in accordance with one or more embodiments;
FIG. 2 illustrates a method to estimate circadian phase of a subject, according to one or more embodiments.

DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, *i.e.,* through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other. As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (*i.e.,* a plurality). As used herein, the term "include" shall be used inclusively to mean any item of a list, by example and without limitation, and/or any combination of items in that list, to the extent possible. Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

Mammalian circadian systems coordinate the timing of an animal's physiological and behavioral functions with local position on the planet. The circadian system depends primarily upon the 24-hour light-dark pattern incident on the retinae. The phototransduction mechanisms responsible for human circadian phototransduction are understood well enough that devices may take advantage of this understanding and adjust circadian timing as desired and/or use it for diagnostic purposes.

Various biomarkers may serve to establish a particular moment in the circadian process, which may be referred to as circadian phase. For example, the time at which the core body temperature attains a minimum value may be a biomarker. This time or moment may be referred to as core body temperature minimum or CBTmin. As used herein, the term CBTmin may be used to indicate the value of the minimum core body temperature or the moment of its attainment, depending on the context of the reference. In some embodiments, CBTmin is used as the zero phase (of the circadian rhythm), the zero-point of the circadian phase, or a circadian phase having value zero. Also the moment at which melatonin production starts, under dim-light conditions - is a so-called biomarker. This moment may be referred to as dim-light melatonin onset or DLMO. In some embodiments, DLMO is used to denote circadian phase. For example, under certain light conditions, DLMO occurs at around 22:30 h. The circadian phase is generally and/or grossly cyclical, repeating itself about every 24 hours.

In some embodiments, models that may be used to determine and/or estimate a subject's circadian phase may use one or more of the following information as input: light exposure during a preceding period, CBTmin, DLMO, subject-specific parameters, sleep-wake information, and/or other types of information. Note that light exposure may shift the circadian phase of a subject, depending on, at least, the intensity of the light and the relative timing of light exposure with respect to the current circadian phase. In some models, multiple types of information may be used in combination. For example, light administered after the CBTmin (typically early in the morning for a properly aligned circadian rhythm) may advance the circadian phase, whereas light administered before the CBTmin (typically in the evening or early in the night for a properly aligned circadian rhythm) may delay the circadian phase. Examples of such models include, but are not limited to, the human circadian pacemaker (HCP) model and its derivatives.

As used herein, the term "determine" (and derivatives thereof) may include measure, calculate, compute, estimate, approximate, generate, and/or otherwise derive, and/or any combination thereof. As used herein, the term "obtain" (and derivatives thereof) may include active and/or passive retrieval, determination, derivation, transfer, upload, download, submission, and/or exchange of information, and/or any combination thereof.

According to the invention, a model is used to determine and/or estimate a subject's circadian phase. The model is described by a second-order differential equation, where the dependent variable $x(t)$ is assumed to vary analogously with a subject's core body temperature (CBT):

$$\frac{d^2}{dt^2}\,x(t) - \mu \cdot (1 - 4x^2)\frac{d}{dt}x(t) + \left(\frac{2\pi}{\tau}\right)^2 x(t) = \frac{d}{dt}(B(t) + N(t))\qquad(2)$$

This model may be referred to as the pacemaker oscillator model. In this equation, $\mu$ (a stiffness factor, *e.g.*, between 0 and 1) and $\tau$ (the intrinsic period) are given model parameters. B(t) and N(t) are the photic driving term B and the non-photic driving term N. B(t) is traditionally obtained through light exposure measurements. N(t) is traditionally obtained through measurements of external stimuli other than light exposure, *e.g.* measurements of an activity level of the subject. Together, B(t) and N(t) form the 'Zeitgeber' term Z(t). The photic term B(t) may be obtained through a light pre-processor that comprises 3 main steps, namely, (1) a non-linear compression of one or more light exposure parameters, (2) a dynamic modeling of retinal saturation over time, (3) a light-sensitivity modulation depending on the circadian phase at which the subject is exposed to light. By way of non-limiting example, see the following references: Kronauer R. E., Forger D. B. and Jewett M. E., "Quantifying Human Circadian Pacemaker Response to brief, extended and repeated light stimuli over the photopic range" in J.Biological Rhythms, Vol. 14(6), pp. 501-516, 1999, (see also the Errata published in 2000), and M. A. St-Hilaire, E. B. Klerman, Sat Bir Khalsa, K. P. Wright Jr., C. A. Czeisler and R. E Kronauer, "Addition of a non-photic component to a light-based mathematical model of the Human Circadian Pacemaker", Journal of Theoretical Biology, Vol. 247, pp. 583-599, 2007.

In some scenarios, subject-specific light exposure information may not be available, not reliable, and/or otherwise not suitable for determining and/or estimating a subject's circadian rhythm. By virtue of the features described in this disclosure, subject-specific information regarding the activity level of a subject may be used to determine and/or estimate a subject's circadian rhythm/phase, in particular in the absence of subject-specific light exposure information.

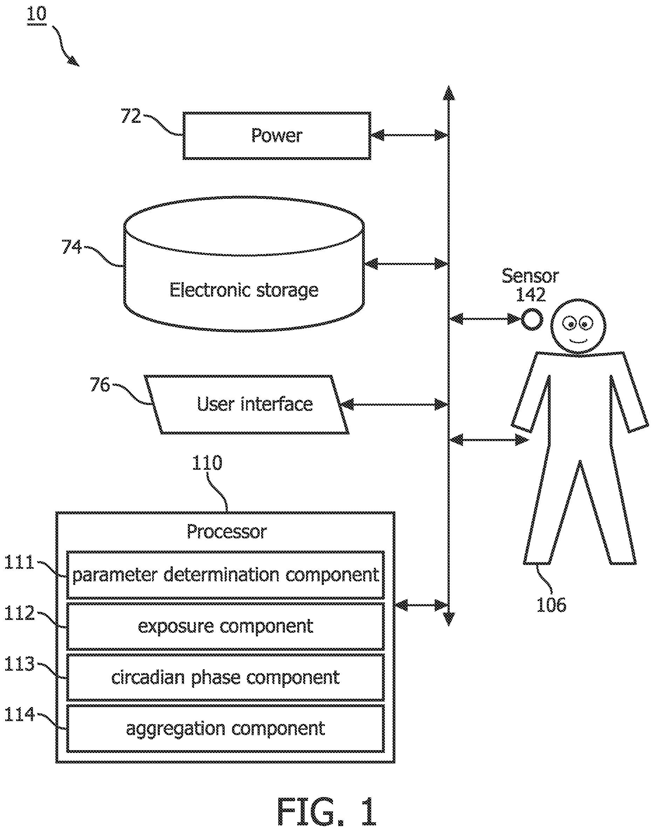

FIG. 1 illustrates a system 10 configured to estimate and/or determine circadian phase of a subject 106, in accordance with one or more embodiments. System 10 includes one or more sensors 142, one or more physical processors 110 and optionally one or more of a power source 72, computer program components, electronic storage 74, a user interface 76, and/or other components. The computer program components include a parameter determination component 111, an exposure component 112, and a circadian phase component 113, and optionally an aggregation component 114, and/or other components.

At least one sensor 142 of system 10 in FIG. 1 is configured to generate output signals conveying information related to an activity level of subject 106. Simultaneously, one or more sensors 142 may be configured to generate output signals conveying information related to energy expenditure by subject 106. Simultaneously, one or more sensors 142 may be configured to generate output signals conveying information related to light exposure of subject 106, physiological, environmental, and/or patient-specific (medical) parameters related to subject 106, and/or other information. System 10 may use any of the generated output signals to monitor subject 106. In some embodiments, the conveyed information may be related to parameters associated with the state and/or condition of subject 106, motion of subject 106, wakefulness and/or sleep state of subject 106, the breathing of subject 106, the gas breathed by subject 106, the heart rate of subject 106, the respiratory rate of subject 106, vital signs of subject 106, including one or more temperatures, oxygen saturation of arterial blood ($SpO_2$), whether peripheral or central, and/or other parameters.

In some embodiments, one or more sensors 142 may generate output signals conveying information related to a location of subject 106, *e.g.* through a gyroscopic sensor in addition to an accelerometer, or GPS technology. The location may be a three-dimensional location of subject 106, a two-dimensional location of subject 106, a location of a specific body part of subject 106 (*e.g.,* eyes, arms, legs, a face, a head, a forehead, and/or other anatomical parts of subject 106), the posture of subject 106, the orientation of subject 106 or one or more anatomical parts of subject 106, and/or other locations.

Sensors 142 include a motion sensor and optionally one or more of an accelerometer, a gyroscopic sensor, a light sensor, an optical sensor, a temperature sensor, a pressure sensor, a weight sensor, an electromagnetic (EM) sensor, an infra-red (IR) sensor, a microphone, a transducer, a heart-rate sensor, a still-image camera, a video camera, and/or other sensors and combinations thereof.

The illustration of sensor 142 including one member in FIG. 1 is not intended to be limiting. System 10 includes one or more sensors. The illustration of a particular symbol or icon for sensor 142 in FIG. 1 is exemplary and not intended to be limiting in any way. The illustration of sensor 142 in a particular location or spatial relation relative to subject 106 in FIG. 1 is exemplary and not intended to be limiting in any way. In some embodiments, one or more sensors 142 may be embedded in an article that is carried by or worn by subject 106, including but not limited to a watch, footwear, apparel, and/or other articles. Resulting signals or information from one or more sensors 142 may be transmitted to processor 110, user interface 76, electronic storage 74, and/or other components of system 10. This transmission can be wired and/or wireless.

One or more sensors 142 may be configured to generate output signals in an ongoing manner, *e.g.* throughout the day, week, month, and/or years. This may include generating signals intermittently, periodically (*e.g.* at a sampling rate), continuously, continually, at varying intervals, and/or in other ways that are ongoing during at least a portion of period of a day, week, month, or other duration. The sampling rate may be about 0.001 second, 0.01 second, 0.1 second, 1 second, about 10 seconds, about 1 minute, about 2 minutes, about 3 minutes, about 4 minutes, about 5 minutes, about 10 minutes, about 15 minutes, about 30 minutes, and/or other sampling rates. In some embodiments, the sampling rate may be any sampling rate between 0.01 and 100 Hz. It is noted that multiple individual sensors may operate using different sampling rates, as appropriate for the particular output signals and/or (frequencies related to particular) parameters derived therefrom. For example, in some embodiments, the generated output signals may be considered as a set of output signals, an ordered set of output signals, a sequence of output signals, and/or a vector of output signals, such that multiple measurements and/or samples of information are conveyed. A particular parameter determined in an ongoing manner from multiple output signals may be considered as a vector of that particular parameter. In some embodiments, system 10 may include two or more sensors 142, *e.g.* two or more accelerometers.

Physical processor 110 (interchangeably referred to herein as processor 110) is configured to provide information processing and/or system control capabilities in system 10. As such, processor 110 may include one or more of a digital processor, an analog processor, a digital circuit designed to process information, an analog circuit designed to process information, and/or other mechanisms for electronically processing information. In order to provide the functionality attributed to processor 110 herein, processor 110 may execute one or more components. The one or more components may be implemented in software; hardware; firmware; some combination of software, hardware, and/or firmware; and/or otherwise implemented. Although processor 110 is shown in FIG. 1 as a single entity, this is for illustrative purposes only. In some implementations, processor 110 may include a plurality of processing units. These processing units may be physically located within the same device, or processor 110 may represent processing functionality of a plurality of devices operating in coordination.

As is shown in FIG. 1, processor 110 is configured to execute parameter determination component 111, an exposure component 112, and a circadian phase component 113, and optionally an aggregation component 114, and/or other components. Processor 110 may be configured to execute components 111-114 by software; hardware; firmware; some combination of software, hardware, and/or firmware; and/or other mechanisms for configuring processing capabilities on processor 110.

It should be appreciated that although components 111-114 are illustrated in FIG. 1 as being co-located within a single processing unit, in implementations in which processor 110 includes multiple processing units, one or more of components 111-114 may be located remotely from the other components. The description of the functionality provided by the different components 111-114 described below is for illustrative purposes, and is not intended to be limiting, as any of components 111-114 may provide more or less functionality than is described. For example, one or more of components 111-114 may be eliminated, and some or all of its functionality may be provided by other ones of components 111-114. Note that processor 110 may be configured to execute one or more additional components that may perform some or all of the functionality attributed below to one of components 111-114.

Parameter determination component 111 of system 10, depicted in FIG. 1, is configured to determine activity parameters and optionally one or more of the following types of parameters: energy expenditure parameters, light exposure parameters, status parameters, medical parameters, and/or other parameters from output signals generated by one or more sensors 142. Parameters may be related to a subject's physiological, environmental, and/or patient-specific parameters. One or more medical parameters may be related to monitored vital signs of subject 106, and/or other medical parameters of subject 106. For example, one or more medical parameters may be related to whether subject 106 is awake or asleep, or, in particular, what the current sleep stage of subject 106 is. Other parameters may be related to the environment near system 10 and/or near subject 106, such as, *e.g.,* air temperature, ambient noise level, ambient light level, and/or other environmental parameters. In some embodiments, an activity parameter may represent an amount of movement by subject 106 (*e.g.* physical movement), for example during a predetermined amount of time (*e.g.* about 1 second, about 10 seconds, about 30 seconds, about 1 minute, about 2 minutes, about 3 minutes, about 4 minutes, about 5 minutes, about 10 minutes, about 15 minutes, about 20 minutes, about 30 minutes, about 1 hour, and/or another period of time). In some embodiments, the predetermined amount of time may be any duration between 1 second and 1 hour. In some embodiments, individual activity parameters may be based on individual generated output signals. In some embodiments, individual activity parameters may be based on multiple generated output signals, *e.g.* by aggregating multiple generated output signals measured at different moments in time, and/or measured by different sensors 142. In some embodiments, parameter determination component 111 may be configured to process and/or pre-process generated output signals that are used to determine a parameter. For example, generated output signals may be averaged, smoothed, clipped at a low or minimum threshold, clipped at a high or maximum threshold, and/or otherwise processed.

One or more physiological parameters may be related to and/or derived from electro-encephalogram (EEG) measurements, electromyogram (EMG) measurements, respiration measurements, cardiovascular measurements, heart-rate-variability (HRV) measurements, autonomic nervous system (ANS) measurements, and/or other measurements. Some or all of this functionality may be incorporated or integrated into other computer program components of processor 110.

In some embodiments, parameter determination component 111 may be configured to determine, track, and/or monitor one or more parameters during a period spanning minutes, hours, days, and/or weeks. For example, in some embodiments, parameter determination component 111 may be configured to determine a light exposure parameter, based on output signals generated by one or more sensors 142, during a period spanning at least 24 hours, and/or intermittently, periodically (*e.g.* at a sampling rate), continuously, continually, at varying intervals, and/or in other ways that are ongoing during at least a day, at least 24 hours, at least 2 days, at least 3 days, at least 4 days, at least 5 days, at least a week, about 2 weeks, about 3 weeks, about 4 weeks, about a month, about two months, or other duration. For example, parameter determination component 111 may be configured to determine a vector of light exposure parameters.

Exposure component 112 is configured to generate one or more light exposure parameters, including but not limited to estimated light exposure parameters. A light exposure parameter may be the amount of light that a person and/or one or more sensors have been exposed to (and/or would have been exposed to) in a predetermined period of time. For example, a light exposure parameter may represent the amount of light that has been received by (and/or that would have been received by) a light sensor and/or light meter.

According to the invention, exposure component 112 is configured to generate a set of estimated light exposure parameters based on one or more activity parameters and optionally other parameters (*e.g.* as determined by parameter determination component 111). In some embodiments, individual estimated light exposure parameters may be based on individual activity parameters. In some embodiments, individual estimated light exposure parameters may be based on multiple activity parameters, *e.g.* by aggregating multiple activity parameters. In some embodiments, exposure component 112 may be configured to process and/or pre-process parameters that are used to generate a light exposure parameter. For example, activity parameters may be averaged, smoothed, clipped at a low or minimum threshold, clipped at a high or maximum threshold, and/or otherwise processed.

According to the invention, an estimated light exposure parameter LE is described by the following equation, where $K$, $c_0$, and $\gamma$ are constants, and *activity(t)* is an activity parameter that represents an activity level of a subject. For example, these constants may depend on characteristics of sensor 142 (*e.g.* an accelerometer).

$$LE(t) = \frac{1}{K} \cdot \left(activity(t)\right)^{\gamma} - c_0, \qquad 1 < \gamma < 3 \quad (1)$$

In some embodiments, the resulting set of estimated light exposure parameters LE(t) may be constrained to positive values. In some embodiments, a derived set of estimated light exposure parameters LE'(t) may be derived from LE(t). For example, LE'(t) may be averaged, smoothed, clipped at a low or minimum threshold, clipped at a high or maximum threshold, and/or otherwise processed. For example, a maximum threshold for a value of estimated light exposure may be a value that corresponds to direct sunlight, *e.g.* 50.000 lux. In some embodiments, individual values of LE'(t) may be based on measurements and/or generated output signals spanning at least a minute of a subject's activity level, at least 5 minutes, at least 10 minutes, at least 15 minutes, at least 20 minutes, at least 30 minutes, and/or another suitable period and/or duration. For example, in some embodiments, smoothing of LE(t) values to produce LE'(t) values may be accomplished through a moving average filter, and/or other filters.

Circadian phase component 113 is configured to determine and/or estimate circadian phases of subjects, e.g. subject 106. According to the invention, operation of circadian phase component 113 is based on a model for circadian phase, including, but not limited to the models described herein that involve core body temperature (*e.g.* CBTmin), dim-light melatonin onset (DLMO), and/or other biomarkers, physiological parameters, and/or environmental parameters.

According to the invention, operation of circadian phase component 113 is based on a pacemaker oscillator model. According to the invention, at least the photic driving term B and optionally the non-photic driving term N are based on estimated light exposure parameters, including but not limited to LE(t) and optionally LE'(t) as described elsewhere in this disclosure. In some embodiments, values of estimated light exposure parameters LE'(t) may substitute and/or replace values of photic driving term B(t). Alternatively, and/or simultaneously, values of non-photic driving term N(t) may be discarded, removed, ignored, and/or otherwise rendered ineffectual for the operation of circadian phase component 113. In some embodiments, the second-order differential equation may be integrated numerically step by step over time, from an initial time (corresponding to an initial activity parameter $x_0$) and a discrete time step. The circadian phase may be estimated as the time of the minimum core body temperature. In some embodiments, the circadian phase may be estimated as being offset by a predetermined duration from the time of the minimum core body temperature. In some embodiments, the predetermined duration of this offset may be subject-specific, *e.g.* based on a subject's chronotype and/or based on prior measurements. For example, the predetermined duration of this offset may be about 30 minutes, about 45 minutes, about 1 hour, about 75 minutes, about 90 minutes, and/or another suitable duration for the offset used to determine the circadian phase from the time of the minimum core body temperature based on a pacemaker oscillator model in which photic driving term B(t) has been replaced and/or substituted by estimated light exposure parameters. Operation of phase component 112 may be based on one or more parameters, including but not limited to parameters determined and/or generated by computer program components described herein.

In some embodiments, operation of one or more computer program components may be based on seasonal information, dusk and dawn information, geographical information, global positioning information, weather information, forecasts and/or predictions, subject-specific travel plans, subject-specific calendar information, and/or other information.

Aggregation component 114 may be configured to aggregate and/or otherwise process multiple activity parameters into fewer values. For example, aggregation component 114 may be configured such that individual values of an estimated light exposure parameter are based on an aggregation of multiple activity parameters, e.g. from a set of activity parameters. In some embodiments, aggregation component 114 may be configured to aggregate and/or otherwise process multiple parameters and/or output signals into fewer parameters and/or output signals.

Power source 72 provides the power to operate one or more components of system 10. Power source 72 may include a portable source of power (*e.g.,* a battery, a fuel cell, *etc.*), and/or a non-portable source of power (*e.g.,* a wall socket, a large generator, *etc.*). In one embodiment, power source 72 includes a portable power source that is rechargeable. In one embodiment, power source 72 includes both a portable and non-portable source of power, and the subject may be able to select which source of power should be used to provide power to system 10.

Electronic storage 74 includes electronic storage media that electronically store information. The electronic storage media of electronic storage 74 may include one or both of system storage that is provided integrally (*i.e.,* substantially non-removable) with system 10 and/or removable storage that is removably connectable to system 10 via, for example, a port (*e.g.,* a USB port, a FireWire port, *etc.*) or a drive (*e.g.,* a disk drive, *etc.*). Electronic storage 74 may include one or more of optically readable storage media (*e.g.,* optical disks, *etc.*), magnetically readable storage media (*e.g.,* magnetic tape, magnetic hard drive, floppy drive, *etc.*), electrical charge-based storage media (*e.g.,* EPROM, EEPROM, RAM, *etc.*), solid-state storage media (*e.g.,* flash drive, *etc.*), and/or other electronically readable storage media. Electronic storage 74 may store software algorithms, information determined by processor 110, information received via user interface 76, and/or other information that enables system 10 to function properly. For example, electronic storage 74 may record or store one or more activity parameters (as discussed elsewhere herein), one or more models, representations and/or implementations of one or more models, and/or other information. Electronic storage 74 may be a separate component within system 10, or electronic storage 74 may be provided integrally with one or more other components of system 10 (*e.g.,* processor 110).

User interface 76 is configured to provide an interface between system 10 and a user (or medical professional, or other device, or other system) through which the user can provide and/or receive information. This enables data, results, and/or instructions and any other communicable items, collectively referred to as "information," to be communicated between the user and system 10. An example of information that may be conveyed to a subject is the current time, a current activity level, an estimated circadian phase, a scheduled wake-up time, or a scheduled light therapy/treatment. Other examples of information that may be conveyed are: circadian rhythm related information like phase and/or intensity, or user performance related information like activity level, scheduled physical events, and/or mental performance events. Examples of interface devices suitable for inclusion in user interface 76 include a keypad, buttons, switches, a keyboard, knobs, levers, a display screen, a touch screen, speakers, a microphone, an indicator light, an audible alarm, and a printer. Information may be provided to the subject by user interface 76 in the form of auditory signals, visual signals, tactile signals, and/or other sensory signals.

By way of non-limiting example, user interface 76 may include a light source capable of emitting light. The light source may include, for example, one or more of at least one LED, at least one light bulb, a display screen, and/or other sources. User interface 76 may control the light source to emit light in a manner that conveys to the subject information related to operation of system 10. Note that subject 106 and the user of system 10 may be one and the same person.

It is to be understood that other communication techniques, either hardwired or wireless, are also contemplated herein as user interface 76. For example, in one embodiment, user interface 76 may be integrated with a removable storage interface provided by electronic storage 74. In this example, information is loaded into system 10 from removable storage (*e.g.,* a smart card, a flash drive, a removable disk, *etc.*) that enables the user(s) to customize the implementation of system 10. Other exemplary input devices and techniques adapted for use with system 10 as user interface 76 include, but are not limited to, an RS-232 port, RF link, an IR link, modem (telephone, cable, Ethernet, internet or other). In short, any technique for communicating information with system 10 is contemplated as user interface 76

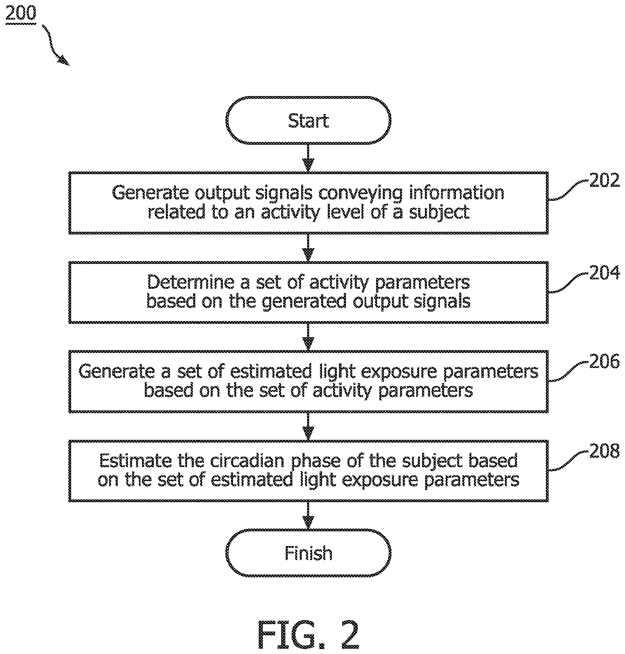

FIG. 2 illustrates a method 200 for estimating circadian phase of subject 106. The operations of method 200 presented below are intended to be illustrative. In some embodiments, method 200 may be accomplished with one or more additional operations not described, and/or without one or more of the operations discussed. Additionally, the order in which the operations of method 200 are illustrated in FIG. 2 and described below is not intended to be limiting.

In some embodiments, method 200 may be implemented in one or more processing devices (*e.g.,* a digital processor, an analog processor, a digital circuit designed to process information, an analog circuit designed to process information, and/or other mechanisms for electronically processing information). The one or more processing devices may include one or more devices executing some or all of the operations of method 200 in response to instructions stored electronically on an electronic storage medium. The one or more processing devices may include one or more devices configured through hardware, firmware, and/or software to be specifically designed for execution of one or more of the operations of method 200.

At an operation 202, output signals are generated that convey information related to an activity level of the subject. In some embodiments, operation 202 is performed by a sensor the same as or similar to sensor 142 (shown in FIG. 1 and described herein).

At an operation 204, a set of activity parameters related to the activity level of the subject is determined. The set of activity parameters is based on the generated output signals. In some embodiments, operation 204 is performed by a parameter determination component the same as or similar to parameter determination component 111 (shown in FIG. 1 and described herein).

At an operation 206, a set of estimated light exposure parameters is generated based on the set of activity parameters. In some embodiments, operation 206 is performed by an exposure component the same as or similar to exposure component 112 (shown in FIG. 1 and described herein).

At an operation 208, the circadian phase of the subject is estimated based on the set of estimated light exposure parameters. In some embodiments, operation 208 is performed by a circadian phase component the same as or similar to circadian phase component 113 (shown in FIG. 1 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the embodiments have been described in detail for the purpose of illustration based on what is currently considered to be most practical and preferred, it is to be understood that such detail is solely for that purpose and that the disclosure is not limited to the disclosed embodiments, but, on the contrary, is intended to cover modifications that are within the scope of the appended claims. For example, it is to be understood that the present disclosure contemplates that, to the extent possible, one or more features of any embodiment can be combined with one or more features of any other embodiment.

**Claims**

1. A system (10) configured to estimate a circadian phase of a subject (106), the system comprising:

a sensor (142) configured to detect a motion of the subject and adapted to generate output signals conveying information related to an activity level of the subject based on said motion; and
one or more physical processors (110), **characterized in that** the one or more physical processors are configured to:

determine a set of activity parameters related to the activity level of the subject, wherein the set of activity parameters is based on the generated output signals;
generate a set of estimated light exposure parameters based on the set of activity parameters using the equation:

$$LE(t) = 1/K \cdot (activity(t))^{\gamma} - c_0, \qquad 1 < \gamma < 3,$$

wherein LE(t) is an estimated light exposure parameter, activity(t) is an activity parameter and K, $\gamma$ and $c_0$ are constants; and

estimate the circadian phase of the subject based on the set of estimated light exposure parameters, wherein estimation of the circadian phase is based on a model including a set of light exposure parameters, wherein the model is a pacemaker oscillator model, wherein the pacemaker oscillator model is described by a second-order differential equation:

$$d^2/dt^2\, x(t) - \mu \cdot (1 - 4x^2)\, d/dt\, x(t) + (2\pi/\tau)^2\, x(t) = d/dt\, (B(t) + N(t)),$$

wherein x(t) is a dependent variable that is assumed to vary analogously with the subject's core body temperature, $\mu$ is a stiffness factor, $\tau$ is an intrinsic period, B(t) is a photic driving term and N is a non-photic driving term, wherein the photic driving term B(t) includes the set of light exposure parameters, and
wherein estimation of the circadian phase includes a substitution of the set of light exposure parameters included in the model with the generated set of estimated light exposure parameters.

2. The system of claim 1, wherein the one or more physical processors (110) are further configured to aggregate multiple activity parameters from the set of activity parameters such that individual ones of the estimated light exposure parameters are based on an aggregation of multiple activity parameters from the set of activity parameters.

3. The system of claim 1, wherein the sensor (142) includes an accelerometer, wherein individual activity parameters in the set of activity parameters reflect an amount of movement by the subject (106) in a predetermined period of time, and wherein the set of activity parameters is an ordered set of activity parameters that spans a period of at least 24 hours.

4. The system of claim 1, wherein estimation of the circadian phase based on the model comprises replacing one or both of a first set of light exposure parameters and a second set of activity parameters with the generated set of estimated light exposure parameters.

5. A method (200) to estimate circadian phase of a subject (106), the method being implemented in a computer system including a sensor (142) configured to detect a motion of the subject and one or more physical processors (110), the method comprising:

generating (202), by the sensor, output signals conveying information related to an activity level of the subject based on said motion;

and **characterized in that** the method further comprises:

determining (204) a set of activity parameters related to the activity level of the subject, wherein the set of activity parameters is based on the generated output signals;

generating (206) a set of estimated light exposure parameters based on the set of activity parameters using the equation:

$$LE(t) = 1/K \cdot (activity(t))^{\gamma} - c_0, \qquad 1 < \gamma < 3,$$

wherein LE(t) is an estimated light exposure parameter, activity(t) is an activity parameter and K, $\gamma$ and $c_0$ are constants; and

estimating (208) the circadian phase of the subject based on the set of estimated light exposure parameters, wherein estimating the circadian phase is performed based on a model including a set of light exposure parameters, wherein the model is a pacemaker oscillator model, wherein the pacemaker oscillator model is described by a second-order differential equation:

$$d^2/dt^2\, x(t) - \mu \cdot (1 - 4x^2)\, d/dt\, x(t) + (2\pi/\tau)^2\, x(t) = d/dt\, (B(t) + N(t)),$$

wherein x(t) is a dependent variable that is assumed to vary analogously with the subject's core body temperature, $\mu$ is a stiffness factor, $\tau$ is an intrinsic period, B(t) is a photic driving term and N is a non-photic driving term, wherein the photic driving term includes the set of light exposure parameters, and

wherein estimating the circadian phase includes substituting the set of light exposure parameters included in the model with the generated set of estimated light exposure parameters.

6. The method of claim 5, further comprising:
aggregating multiple activity parameters from the set of activity parameters, wherein generating the set of estimated light exposure parameters is performed such that individual ones of the estimated light exposure parameters are based on an aggregation of multiple activity parameters from the set of activity parameters.

7. The method of claim 5, wherein the sensor (142) includes an accelerometer, wherein individual activity parameters in the set of activity parameters reflect an amount of movement by the subject (106) in a predetermined period of time, and wherein the set of activity parameters is an ordered set of activity parameters that spans a period of at least 24 hours.

8. The method of claim 7, wherein estimating the circadian phase based on the model comprises replacing one or both of a first set of light exposure parameters and a second set of activity parameters with the generated set of estimated light exposure parameters.

**Patentansprüche**

1. System (10), das konfiguriert ist, um eine zirkadiane Phase eines Subjekts (106) zu schätzen, das System Folgendes umfassend: einen Sensor (142), der konfiguriert ist, um eine Bewegung des Subjekts zu erfassen, und der angepasst ist, um Ausgangssignale zu erzeugen, die basierend auf der Bewegung Informationen in Bezug auf ein Aktivitätsniveau des Subjekts zu übermitteln; und einen oder mehrere physische Prozessoren (110), **dadurch gekennzeichnet, dass** der eine oder die mehreren physischen Prozessoren zu Folgendem konfiguriert sind: Bestimmen eines Satzes von Aktivitätsparametern, die mit dem Aktivitätsniveau des Subjekts in Beziehung stehen, wobei der Satz von Aktivitätsparametern auf den erzeugten Ausgangssignalen basiert; Erzeugen eines Satzes von geschätzten Lichtexpositionsparametern basierend auf dem Satz von Aktivitätsparametern unter Verwendung der Gleichung:

$$LE(t) = 1/K \cdot (Aktivität(t))^{\gamma} - c_0, \quad 1 < \gamma < 3,$$

wobei LE(t) ein geschätzter Lichtexpositionsparameter ist, Aktivität(t) ein Aktivitätsparameter ist und K, $\gamma$ und $c_0$ Konstanten sind; und Schätzen der zirkadianen Phase des Subjekts basierend auf dem Satz von geschätzten Lichtexpositionsparametern, wobei die Schätzung der zirkadianen Phase auf einem Modell basiert, das einen Satz von Lichtexpositionsparametern beinhaltet, wobei das Modell ein Schrittmacher-Oszillatormodell ist, wobei das Schrittmacher-Oszillatormodell durch eine Differentialgleichung zweiter Ordnung beschrieben ist:

$$d^2/dt^2\ x(t)\ -\ \mu\ \bullet\ (1\ -\ 4x^2)\ d/dt\ x(t)\ +\ (2\pi/\tau)^2\ x(t)\ =$$

$$d/dt\ (B(t)\ +\ N(t)),$$

wobei x(t) eine abhängige Variable ist, von der angenommen wird, dass sie sich analog zur Körperkerntemperatur des Subjekts ändert, μ ein Steifigkeitsfaktor ist, τ eine Eigenperiode ist, B(t) ein photischer Antriebsterm ist und N ein nicht-photischer Antriebsterm ist, wobei der photische Antriebsterm B(t) den Satz von Lichtexpositionsparametern beinhaltet, und wobei eine Schätzung der zirkadianen Phase eine Substitution des im Modell beinhalteten Satzes von Lichtexpositionsparametern durch den erzeugten Satz von geschätzten Lichtexpositionsparametern beinhaltet.

2. System nach Anspruch 1, wobei der eine oder die mehreren physischen Prozessoren (110) ferner konfiguriert sind, um mehrere Aktivitätsparameter aus dem Satz von Aktivitätsparametern zu aggregieren, sodass einzelne der geschätzten Lichtexpositionsparameter auf einer Aggregation mehrerer Aktivitätsparameter aus dem Satz von Aktivitätsparametern basierend.

3. System nach Anspruch 1, wobei der Sensor (142) einen Beschleunigungsmesser beinhaltet, wobei einzelne Aktivitätsparameter in dem Satz von Aktivitätsparametern ein Ausmaß an Bewegung durch das Subjekt (106) in einem vorbestimmten Zeitraum widerspiegeln, und wobei der Satz von Aktivitätsparametern ein geordneter Satz von Aktivitätsparametern ist, der sich über einen Zeitraum von mindestens 24 Stunden erstreckt.

4. System nach Anspruch 1, wobei eine Schätzung der zirkadianen Phase basierend auf dem Modell ein Ersetzen eines ersten Satzes von Lichtexpositionsparametern und eines zweiten Satzes von Aktivitätsparametern durch den erzeugten Satz von geschätzten Lichtexpositionsparametern oder beides umfasst.

5. Verfahren (200) zum Schätzen einer zirkadianen Phase eines Subjekts (106), wobei das Verfahren in einem Computersystem implementiert ist, das einen Sensor (142), der konfiguriert ist, um eine Bewegung des Subjekts zu erfassen, und einen oder mehrere physische Prozessoren (110) beinhaltet, das Verfahren umfassend:

Erzeugen (202), durch den Sensor, von Ausgangssignalen, die basierend auf der Bewegung Informationen in Bezug auf ein Aktivitätsniveau des Subjekts übermitteln; und
**dadurch gekennzeichnet, dass** das Verfahren ferner Folgendes umfasst:

Bestimmen (204) eines Satzes von Aktivitätsparametern, die mit dem Aktivitätsniveau des Subjekts in Beziehung stehen, wobei der Satz von Aktivitätsparametern auf den erzeugten Ausgangssignalen basiert;
Erzeugen (206) eines Satzes von geschätzten Lichtexpositionsparametern basierend auf dem Satz von Aktivitätsparametern unter Verwendung der Gleichung:

$$LE(t)\ =\ 1/K\ \bullet\ (Aktivität(t))^\gamma\ -\ c_0,\ 1\ <\ \gamma\ <\ 3,$$

wobei LE(t) ein geschätzter Lichtexpositionsparameter ist, Aktivität(t) ein Aktivitätsparameter ist und K, γ und $c_0$ Konstanten sind; und Schätzen (208) der zirkadianen Phase des Subjekts basierend auf dem Satz von geschätzten Lichtexpositionsparametern, wobei ein Schätzen der zirkadianen Phase basierend auf einem Modell ausgeführt wird, das einen Satz von Lichtexpositionsparametern beinhaltet, wobei das Modell ein Schrittmacher-Oszillatormodell ist, wobei das Schrittmacher-Oszillatormodell durch eine Differentialgleichung zweiter Ordnung beschrieben ist:

$$d^2/dt^2\ x(t)\ -\ \mu\ \bullet\ (1\ -\ 4x^2)\ d/dt\ x(t)\ +\ (2\pi/\tau)^2\ x(t)\ =$$

$$d/dt\ (B(t)\ +\ N(t)),$$

wobei x(t) eine abhängige Variable ist, von der angenommen wird, dass sie sich analog zur Körperkerntemperatur des Subjekts ändert, μ ein Steifigkeitsfaktor ist, τ eine Eigenperiode ist ein photischer Antriebsterm ist und N ein nicht-photischer Antriebsterm ist, wobei der photische Antriebsterm den Satz von Lichtexpositionsparametern beinhaltet, und wobei ein Schätzen der zirkadianen Phase ein Substituieren des in dem Modell beinhalteten Satzes von Lichtexpositionsparametern durch den erzeugten Satz von geschätzten Lichtexpositionsparametern beinhaltet.

6. Verfahren nach Anspruch 5, ferner umfassend:
Aggregieren mehrerer Aktivitätsparameter aus dem Satz von Aktivitätsparametern, wobei ein Erzeugen des Satzes von geschätzten Lichtexpositionsparametern ausgeführt wird, sodass einzelne der geschätzten Lichtexpositionsparameter auf einer Aggregation von mehreren Aktivitätsparametern aus dem Satz von Aktivitätsparametern basieren.

7. Verfahren nach Anspruch 5, wobei der Sensor (142) einen Beschleunigungsmesser beinhaltet, wobei einzelne Aktivitätsparameter in dem Satz von Aktivitätsparametern ein Ausmaß an Bewegung durch das Subjekt (106) in einem vorbestimmten Zeitraum widerspiegeln, und wobei der Satz von Aktivitätsparametern ein geordneter Satz von Aktivitätsparametern ist, der sich über einen Zeitraum von mindestens 24 Stunden erstreckt.

8. System nach Anspruch 7, wobei ein Schätzen der zirkadianen Phase basierend auf dem Modell ein Ersetzen eines ersten Satzes von Lichtexpositionsparametern und eines zweiten Satzes von Aktivitätsparametern durch den erzeugten Satz von geschätzten Lichtexpositionsparametern oder beides umfasst.

**Revendications**

1. Un système (10) configuré pour estimer une phase circadienne d'un sujet (106), le système comprend:
un capteur (142) configuré pour détecter un mouvement du sujet et adapté pour générer des signaux de sortie transmettant l'information liée à un niveau d'activité du sujet fondé sur ledit mouvement; et un ou plusieurs processeurs physiques (110), **caractérisés par le fait qu'**un ou plusieurs processeurs physiques sont configurés pour :

déterminer un ensemble de paramètres d'activité lié au niveau d'activité du sujet, où l'ensemble de paramètres d'activité est fondé sur les signaux de sortie générés;
produire un ensemble de paramètres d'exposition à la lumière estimés fondés sur l'ensemble de paramètres d'activité utilisant l'équation:

$$\mathrm{LE(t)} = 1/K \cdot (\text{activité}(t))^y - c_0, \quad 1 < y < 3,$$

où LE(t)sont des paramètres d'exposition à la lumière, activité (t) est un paramètre d'activité et K, y et $c_0$ sont constants; et estimer la phase circadienne du sujet fondé sur l'ensemble de paramètres d'exposition à la lumière estimés, où l'estimation de la phase circadienne est fondée sur un modèle comprenant un ensemble de paramètres d'exposition à la lumière où le modèle est un modèle d'oscillateur de stimulateur cardiaque, où le modèle de stimulateur cardiaque est décrit par une équation différentielle de second ordre:

$$d^2/dt^2 \; x(t) - \mu \cdot (1 - 4x^2) \; d/dt \; x(t) + (2\pi/\tau)^2 \; x(t) =$$

$$d/dt \; (B(t) + N(t)),$$

où x(t) est une variable dépendante qui est supposée varier de manière analogue avec la température corporelle centrale du sujet, $\mu$ est un facteur de rigidité, $\tau$ est une période intrinsèque, B(t) est un terme photique de conduite et N est un terme non-photique de conduite, où le terme photique de conduite B(t) inclut l'ensemble de paramètres d'exposition à la lumière, et où l'estimation de la phase circadienne inclut une substitution de l'ensemble des paramètres d'exposition de la lumière inclut dans le modèle avec l'ensemble généré de paramètres d'exposition à la lumière.

2. Le système de la revendication 1, où un ou plusieurs processeurs physiques (110) sont également configurés pour agréger plusieurs paramètres d'activité à partir de l'ensemble de paramètres d'activité de sorte que les paramètres individuels des paramètres d'exposition à la lumière estimée sont fondés sur une agrégation de paramètres d'activité multiple à partir de l'ensemble de paramètres d'activité.

3. Le système de la revendication 1, où le capteur (142) inclut un accéléromètre, où les paramètres d'activité individuelle dans l'ensemble des paramètres d'activité reflètent une quantité de mouvement par le sujet (106) dans une période prédéterminée, et où l'ensemble de paramètres d'activité est un ensemble ordonné de paramètres d'activité qui s'étend sur une période d'au moins 24 heures.

4. Le système de la revendication 1, où l'estimation de la phase circadienne fondée sur le modèle comprend le remplacement d'un ou plusieurs ensembles de paramètres d'exposition à la lumière et un second ensemble de paramètres d'activité avec l'ensemble généré des paramètres d'exposition à la lumière estimée.

5. Une méthode (200) pour estimer la phase circadienne du sujet (106), la méthode étant implémentée dans un système informatique comprenant un capteur (142) configuré pour détecter un mouvement du sujet et un ou plusieurs processeurs physiques (110), la méthode comprend: produire (202), par le capteur, des signaux de sortie transmettant l'information liée au niveau d'activité du sujet fondé sur ledit mouvement; et **caractérisé par le fait que** la méthode comprend également:

la détermination (204) d'un ensemble de paramètres d'activité lié au niveau d'activité du sujet, où l'ensemble de paramètres d'activité est fondé sur les signaux de sortie générés; produire (206) un ensemble de paramètres d'exposition à la lumière estimés fondés sur l'ensemble des paramètres d'activité utilisant l'équation:

$$LE(t) = 1/K \cdot (\text{activité}(t))^Y - c_0, \quad 1 < Y < 3,$$

où LE(t) sont des paramètres d'exposition à la lumière, activité (t) est un paramètre d'activité et K, Y et $c_0$ sont constants; et estimer (208) la phase circadienne du sujet fondé sur l'ensemble des paramètres d'exposition à la lumière estimée, où l'estimation de la phase circadienne est exécutée fondée sur un modèle comprenant un ensemble de paramètres d'exposition à la lumière, où le modèle est un modèle d'oscillateur de stimulateur cardiaque, où le modèle d'oscillateur de stimulateur cardiaque est décrit par une équation différentielle de second ordre:

$$d^2/dt^2 \ x(t) - \mu \cdot (1 - 4x^2) \ d/dt \ x(t) + (2\pi/\tau))^2 \ x(t) =$$

$$d/dt \ (B(t) + N(t)),$$

où x(t) est une variable dépendante qui est supposée varier de façon analogue avec la température corporelle centrale du sujet, $\mu$ est un facteur de rigidité, $\tau$ est une période intrinsèque, B(t) est un terme photique de conduite et N est le terme de conduite non-photique, où le terme de conduite photique inclut l'ensemble de paramètres d'exposition à la lumière, et où l'estimation de la phase circadienne inclut la substitution de l'ensemble de paramètres d'exposition à la lumière inclut dans le modèle avec l'ensemble généré de paramètres d'exposition à la lumière.

6. La méthode de la revendication 5 comprend également: l'agrégation de plusieurs paramètres d'activité de l'ensemble de paramètres d'activité, où la production de l'ensemble de paramètres d'exposition à la lumière est exécutée de sorte que les paramètres des paramètres d'exposition à la lumière estimés sont fondés sur une agrégation de plusieurs paramètres d'activité de l'ensemble des paramètres d'activité.

7. La méthode selon la revendication 5, où le capteur (142) inclut un accéléromètre, où les paramètres d'activité individuels dans l'ensemble de paramètres d'activité reflètent la quantité de mouvement par le sujet (106) dans une période prédéterminée, et où l'ensemble de paramètres d'activité est un ensemble ordonné de paramètres d'activité qui s'étend sur une période d'au moins 24 heures.

8. La méthode selon la revendication 7, où l'estimation de la phase circadienne fondée sur le modèle comprend le remplacement d'un ou plusieurs ensembles de paramètres d'exposition à la lumière et un second ensemble de paramètres d'activité avec l'ensemble généré de paramètres d'exposition à la lumière.

10

72 — Power

74 — Electronic storage

Sensor 142

106

76 — User interface

110

Processor

111 — parameter determination component

112 — exposure component

113 — circadian phase component

114 — aggregation component

FIG. 1

200

Start

Generate output signals conveying information
related to an activity level of a subject — 202

Determine a set of activity parameters
based on the generated output signals — 204

Generate a set of estimated light exposure parameters
based on the set of activity parameters — 206

Estimate the circadian phase of the subject based
on the set of estimated light exposure parameters — 208

Finish

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013132454 A

**Non-patent literature cited in the description**

- **SONIA ANCOLI-ISRAEL et al.** The role of actigraphy in the study of sleep and circadian rhythms. *SLEEP,* 01 May 2003, vol. 26 (3), 342-392
- **KRONAUER R. E. ; FORGER D. B. ; JEWETT M. E.** Quantifying Human Circadian Pacemaker Response to brief, extended and repeated light stimuli over the photopic range. *J.Biological Rhythms,* 1999, vol. 14 (6), 501-516
- *Errata,* 2000
- **M. A. ST-HILAIRE ; E. B. KLERMAN ; SAT BIR KHALSA ; K. P. WRIGHT JR. ; C. A. CZEISLER ; R. E KRONAUER.** Addition of a non-photic component to a light-based mathematical model of the Human Circadian Pacemaker. *Journal of Theoretical Biology,* 2007, vol. 247, 583-599